Europäisches Patentamt

European Patent Office

Office européen des brevets

(11) Publication number: **0 199 518 B1**

# EUROPEAN PATENT SPECIFICATION

(45) Date of publication of patent specification: **13.11.91** (51) Int. Cl.⁵: **B01D 63/02**, A61L 2/06

(21) Application number: 86302747.0

(22) Date of filing: 14.04.86

(54) Steam sterilisation of a filtration device.

(30) Priority: 18.04.85 JP 81347/85
18.04.85 JP 81348/85
21.05.85 JP 107063/85

(43) Date of publication of application:
29.10.86 Bulletin 86/44

(45) Publication of the grant of the patent:
13.11.91 Bulletin 91/46

(84) Designated Contracting States:
CH DE FR GB IT LI

(56) References cited:
FR-A- 2 525 488
GB-A- 1 575 377

PATENT ABSTRACTS OF JAPAN, vol. 10, no.
144 (C-349)[2201], 27th May 1986; & JP-A-61
4509 (KOGYO GIJUTSUIN (JAPAN))
10-01-1986

PATENT ABSTRACTS OF JAPAN, vol. 6, no.
266 (C-142)[1144], 25th December 1982; &
JP-A-57 159 502 (TOYO BOSEKI K.K.)
01-10-1982

PATENT ABSTRACTS OF JAPAN, vol. 7, no. 3
(C-143)[1148], 7th January 1983; & JP-A-57

160 943 (TOYO BOSEKI K.K.) 04-10-1982

ENCYCLOPEDIA OF POLYMER SCIENCE AND
ENGINEERING, 2nd edition, by M. Menges,
Bikales and Overberger, vol. 11, p.656, table
1

(73) Proprietor: **Asahi Kasei Kogyo Kabushiki
Kaisha
2-6, Dojimahama 1-chome Kita-ku
Osaka-shi Osaka 530(JP)**

(72) Inventor: **Nejigaki, Tatuo
14-5 Motocho
Fuji-shi Shizuoka-ken(JP)**
Inventor: **Kakutani, Yoshimi
RM B-43 Endou-Apartment 68 Aoshima
Funi-ahi Shizuoka-ken(JP)**

(74) Representative: **Woodcraft, David Charles et
al
BROOKES & MARTIN High Holborn House
52/54 High Holborn
London, WC1V 6SE(GB)**

## Description

Field of the Invention

This invention relates to a process for sterilizing a filtration device, and more particularly to a process for sterilizing a filtration device having a hollow fiber type module with steam.

Description of the Prior Art

As a method for sterilizing a semipermeable membrane filtration device, the method by use of chemicals such as sodium hypochlorite or the heating sterilization method with hot water of about 90 °C have been employed.

As the method with the use of a chemicals, there has been employed the sterilization method in which a sterilizer such as sodium hypochlorite, hydrogen peroxide and formalin etc. is filtered through the semipermeable membrane in the semipermeable membrane filtration device from the raw water side. However, according to this method, if there remains air within the pipelines due to the structures of pipelines or instruments, some portions remain unfilled with the sterilizer to be insufficiently sterilized at that portions.

On the other hand, according to the hot water sterilization, even when sterilization was effected with hot water of about 90 °C, heat resistant spores which cannot be killed at about 90 °C may sometimes exist, or there may be air or dead space remaining in the filtrate pipelines, whereby such portions can be replaced with hot water with difficulty to give only insufficient sterilizing effect.

Further, after sterilization was performed with a sterilizer as described above, it is necessary to wash out the sterilizer and for this purpose thorough rinsing with water is required to be performed, whereby not only a long time is taken, but also sometimes its waste water must be disposed of.

In the prior art, there has been no filtration device by use of a hollow fiber type membrane module which can withstand internal steam sterilization. This is because the hollow fiber type module employs generally hollow fiber membranes fixed at both ends onto a module case with a sealing material, whereby the membrane properties may be lowered under high temperature and at high pressure conditions during steam sterilization or seal leak may occur at the sealed portions.

GB-A-1575377 discloses an artificial kidney device which comprises a bundle of hollow fiber membranes whose ends are sealed to each other and to the case of the device and which uses heat resistant materials that allow the device to be sterilized by externally applied steam. A pressure buffer is provided to receive primer liquid contained within the device which expands during the sterilization.

## SUMMARY OF THE INVENTION

The present invention is based on the selection of heat property parameters for a hollow fiber filtration device which allow a sterilization technique where steam is passed internally through the device. As a result the present invention provides a process for sterilizing a filtration device which requires no rinsing with water after sterilization and yet can sterilize at a high temperature without fear of secondary contamination.

This invention provides a process for sterilizing a filtration device which comprises (a) providing a module case containing a bundle of hollow fibers of heat resistant polymer with a heat distortion temperature of $70\,°C$ or higher under a load of $182 \times 104$ Pa ($18.6$ kg/cm$^2$) and with pore diameter of less than $0.45\,\mu$m, the fibres being sealed together and to the inner surfaces of the case adjacent to each end of the bundle by a sealing material such that the difference in average linear expansion coefficient between the sealing material and the module case at temperature of $20 - 121\,°C$ is $7 \times 10^{-5}/°C$ or lower; (b) passing steam from the raw water side of the module to the filtrate side thereof, while withdrawing all or part of the raw water and filtrate in the module as residue of the filtration cycle or alternatively after withdrawal of said raw water and filtrate.

## BRIEF DESCRIPTION OF THE DRAWINGS

FIG. 1 shows the structure of a typical hollow fiber type module which can be used in this invention.
FIG. 2 shows an outline of a typical filtration device in which the hollow fiber type module is used.

## DETAILED DESCRIPTION OF THE INVENTION

The filtration device which is employed in this invention has a hollow fiber type module for ultrafiltration or microfiltration.

The hollow fiber membrane which can be employed in the module is made from a heat resistant polymer having a heat distortion temperature of 70 °C or higher under the load of $182 \times 10^4$ Pa (18.6 kg/cm$^2$). The heat distortion temperature is measured according to ASTM D648. If the heat distortion temperature is lower than 70 °C, the membrane will be deformed during steam sterilization, whereby the membrane properties may be deteriorated or the membrane may be destroyed to make it no longer useable. The membrane material may be any of materials having a heat distortion temperature of 70 °C or higher under the load of $182 \times 10^4$ Pa (18.6 kg/cm$^2$), including, for example, polyvinylidene fluoride, polypropylene, polyphenylene oxide, ethylene-tetrafluoroethylene copolymer, polysulfone, polyethersulfone and others.

Further, the hollow fiber membrane used in the module has pore diameter of less than 0.45 $\mu$m. The pore diameter is defined as the maximum particle size which can pass through the membrane. The membrane whose pore diameter is 0.45 $\mu$m or more cannot be employed in this invention because fungi can easily pass through the membrane. The pore diameter of 0.2 $\mu$m to 1 nm is preferred.

The hollow fiber membrane which is used in this invention can be prepared according to various methods. As the method for preparation of the membrane, it is possible to use the methods used for preparation of ultrafiltration membranes and microfilters generally known in the art such as the micro-phase separation method, the stretching method and the extraction method.

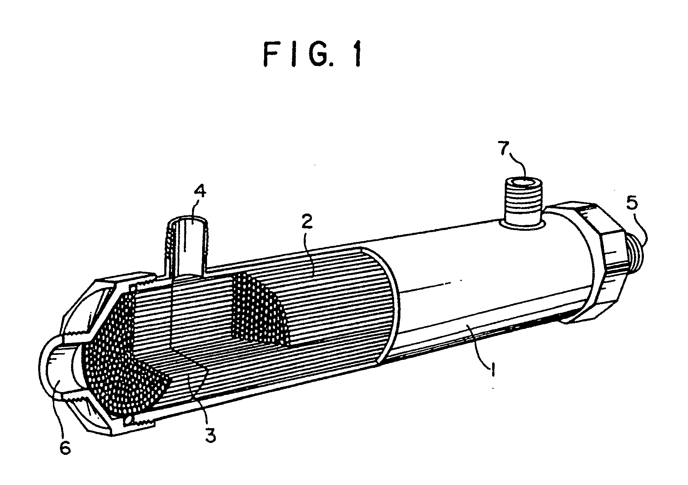

Exemplary hollow fiber type module which can be used in this invention is shown in FIG. 1. The module comprises a module case 1, plurality of hollow fibers 2 and the sealing material 3 for fixing the end portions of hollow fibers 2 in the case 1. 4 is a filtrate outlet, 5 is a raw water inlet, 6 is a concentrated raw water outlet and a steam inlet and 7 is a condensed water outlet. The average linear expansion coefficient between the sealing material and the module case at between 20 °C and 121 °C must be $7 \times 10^{-5}$/°C or lower. If the average linear expansion coefficient is more than $7 \times 10^{-5}$/°C, seal leak will occur at the end portions when the module mounted on the filtration device is subjected to in-line sterilization with steam of 121 °C.

The average linear expansion coefficient ($\beta$) between 20 °C and 121 °C is defined as follows:

$$\beta \ [1/\overset{o}{C}] = \frac{1}{\ell_o} \cdot \frac{\ell_{121} - \ell_{20}}{121 - 20}$$

$$\left( \begin{array}{l} \text{wherein } \ell_o, \ \ell_{20} \text{ and } \ell_{121} \text{ are length} \\ \text{at } 0 \ ^oC, \ 20 \ ^oC \text{ and } 121 \ ^oC, \text{ respectively.} \end{array} \right)$$

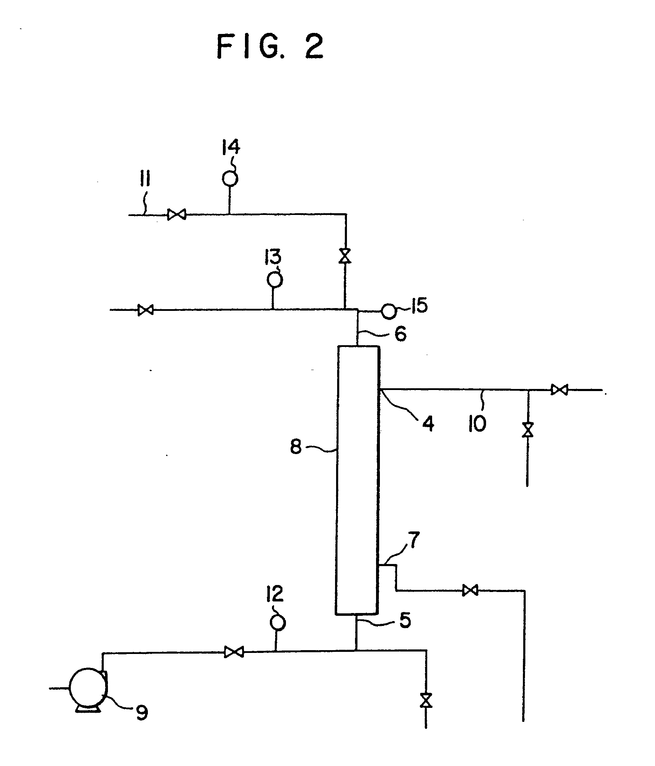

This hollow fiber type module was mounted on a filtration device for removal of microorganisms from raw water as shown in FIG. 2. 8 is a hollow fiber type module, 9 is a feed pump of raw water, 10 is a filtrate pipeline, 11 is a steam feeding pipeline, 12, 13 and 14 are manometers and 15 is a thermometer.

The filtration device having the hollow fiber type module is sterilized by passing steam from the raw water side of the module to the filtrate side thereof, while withdrawing all or a part of the raw water and filtrate in the module or alternatively after withdrawal of raw water and filtrate.

As the steam, a high temperature steam is generally used for enhancing the sterilization effect, but a steam of 121 °C ~ 125 °C is usually employed and a steam of 121 °C is preferred. The sterilization is usually conducted for 0.5 to one hour. The steam pressure which can be applied ranges from 9.8 to $14.7 \times 10^4$ Pa (1 to 1.5 kg/cm$^2$ G).

The steam sterilization of this invention can be carried out at any time, for example, before filtration, on the way of filtration and after filtration.

After passage of steam, the pressure at the sterilized portion becomes lowered due to condensation of steam. Accordingly, if the device is left to stand as such, the pressure will become lower than the atomospheric pressure, whereby there has been involved the problem that the outer air not sterilized may be leaked into the system through the joint of pipelines and the sterilization effect cannot be maintained.

To maintain the sterilization effect, especially on the filtrate side of the device, it is necessary to prevent the filtrate side from becoming negative pressure in this invention. As the method for prevention, the method in which water is introduced into the filtrate side through the membrane from the raw water side to fill the filtrate side with water can be employed. The water used for prevention of negative pressure preferably has a temperature of 80 °C or higher for alleviating the thermal shock on the hollow fiber type module.

Further, the method in which a sterilized gas is filled into the filtrate side after passage of steam can be also employed. The pressure of the gas introduced may be less than $9.8 \times 10^4$ Pa (1 kg/cm²) and preferably be around $4.9 \times 10^4$ Pa (0.5 kg/cm²). Under a pressure of $9.8 \times 10^4$ Pa (1 kg/cm²) or higher, the performances of the filtration membranes will be lowered. As the gas to be introduced, there may be employed air, nitrogen, oxygen, helium and others.

According to the steam sterilization process of the present invention, it has become possible to sterilize internally the filtration device with steam of high temperature. Therefore, complicated steps such as rinsing with water after sterilization or disposal of the chemical solution after a sterilization as required in the prior art methods by use of a sterilizer are no longer necessary. Further, since the steam of high temperature used for sterilization is passed from the raw water side to the filtrate side through the hollow fiber membrane, sterilization can be effected surely on the filtrate side including pipelines connected with the module.

Further, surer sterilization can be practiced as compared with heat sterilization with hot water of the prior art because the module used in this invention has high heat resistance.

Further, because the filtrate side is prevented from becoming a reduced pressure with hot water or a sterilized gas after steam sterilization, whereby the sterilized state can be maintained without contamination with the outer air.

The following examples illustrate the present invention in more detail, but they are given for illustrative purposes only and are not to be construed as limiting the invention.

The number of living microorganisms in the filtrate in Examples was counted by the following method.

1000 ml of filtrate was filtered by Bacteriological Monitor (Trade name; MILLIPORE Corp.). Then, M-TGE medium was fed to Bacteriological Monitor and cultivation was carried out at 33 °C for 48 hours. The number of colonies on the surface of a filter of the Monitor was counted as the number of living microorganisms in 1000 ml of the filtrate.

Example 1

As a hollow fiber type module, the module having about 2400 polysulfone hollow fiber membranes for ultrafiltration with pore diameter of 5 nm, which were fixed in a polysulfone case at the end portions thereof with a sealing material of an epoxy resin was employed. The heat distortion temperature under $182 \times 10^4$ Pa (18.6 kg/cm²) of the polysulfone resin constituting the hollow fiber membranes was 174 °C. The average linear expansion coefficients at temperatures between 20 °C and 121 °C of the module case and the sealing material were $7 \times 10^{-5}/°C$ and $13 \times 10^{-5}/°C$, respectively, and the difference between both was $6 \times 10^{-5}/°C$.

Filtration was performed for two weeks in an ordinary manner by means of a filtration device mounting the module and then steam sterilization was applied to the device. The process of steam sterilization consisted of first stopping the feed pump 9, and introducing steam of $11.8 \times 10^4$ Pa (1.2 kg/cm²) from the steam inlet 6 while discharging the condensed water from the condensed water outlet 7, the filtrate outlet 4 and the raw water inlet 5, thereby filling the module and filtrate pipelines with steam.

After this steam sterilization was carried out at about 121 °C for 30 minutes, feeding of steam was discontinued. Then, before the filtrate side becomes negative pressure, hot water heated to 80 °C was introduced from the raw water inlet to fill the filtrate side with hot water.

After this steam sterilization operation, filtration was performed again in a usual manner, but no seal leak of the module was found to be generated. Also, no living microorganism was found in 1000 ml of the filtrate after running for 2 weeks after the steam sterilization.

Example 2

The same module as Example 1 except that microfilters of ethylene-tetrafluoroethylene copolymer (ETFE) having pore diameter of 0.1 μm were used as a hollow fiber membranes was prepared. The heat distortion temperature under 182x10⁴ Pa (18.6 kg/cm²)of ETFE was 74 °C. The steam sterilization of the filtration device mounting the module was conducted in the same manner as Example 1.

After the steam sterilization, filtration was carried out in a usual manner. Two weeks after the steam sterilization, no living microorganism was found at all in 1000 ml of the filtrate. Also, no seal leak was generated in the module.

Example 3

By using the same filtration device as Example 1 except that a sterilized air feeding means was equipped, filtration was carried out for two weeks in an ordinary manner. Then, the steam sterilization was applied to the device in the same manner as Example 1. After steam sterilization was carried out at about 121 °C for 30 minutes, feeding of steam was discontinued and, before the filtrate side becomes negative pressure, air was introduced from a part of filtrate pipelines to the filtrate side through a filter having a pore diameter of 0.2 μm for removal of microorganisms from the air at a pressure of 4.9x10⁴ Pa (0.5 kg/cm²) until the filtrate side becomes 100 °C or lower. Then, a feeding of air was stopped and raw water was introduced from the raw water inlet to the module. The raw water was passed through membranes to replace the air in the filtrate side with filtrate, and again filtration running was started. The living microorganism in 1000 ml of the filtrate after two weeks after start-up of running was found to be zero, and there was no seal leak at the sealed portions of hollow fiber membrances.

When negative pressure prevention was practiced similarly by changing the pressure of the air to 9.8x10⁴ Pa (1 kg/cm²), there was observed a phenomenon that the water permeability of the hollow fiber membranes in the subsequent usual running was lowered by 5 %, while it was within 1 % when negative pressure prevention was practiced with the use of a pressurized air of 4.9x10⁴ Pa (0.5 kg/cm²).

Comparative Example

The same hollow fiber type module as Example 1 except that a stainless steel module case was used was prepared. The average linear expansion coefficients at temperatures between 20 °C and 121 °C of the module case and the epoxy resin were 2 x 10⁻⁵/°C and 13 x 10⁻⁵/°C, with its difference being 11 x 10⁻⁵/°C. By employing the filtration device mounting this module, the same filtration and steam sterilization as Example 1 were carried out.

After the steam sterilization, filtration was performed again. Two weeks after the steam sterilization, the number of living microorganisms in the filtrate was 830 per 1000 ml and seal leak was found to be generated as judged by the pressurized air test under 9.8x10⁴ Pa (1 kg/cm²).

Example 4

The same module as Example 1 except that an epoxy resin containing 33 parts by weight of silica powder was used as a sealing material was prepared. The average linear expansion coefficients at temperatures between 20 °C and 121 °C of the module case and the sealing material were 7 x 10⁻⁵/°C and 8 x 10⁻⁵/°C, respectively, and the difference between both was 1 x 10⁻⁵/°C.

By using the filtration device mounting the module, filtration and steam sterilization were carried out in the same manner as Example 1.

After the steam sterilization, filtration was carried out again. Two weeks after the steam sterilization, the number of living microorganisms in 1000 ml of the filtrate was zero, and no seal leak was found in the module.

**Claims**

1. A process for sterilizing a filtration device which comprises (a) providing a module case containing a bundle of hollow fibers of heat resistant polymer with a heat distortion temperature of 70° C or higher under a load of 182 x 10⁴ Pa (18.6 kg/cm²) and with pore diameter of less than 0.45 μm, the fibres being sealed together and to the inner surfaces of the case adjacent to each end of the bundle by a sealing material such that the difference in average linear expansion coefficient between the sealing material and the module case at temperature of 20 - 121° C is 7 x 10⁻⁵/° C or lower; (b) passing steam from the raw water side of the module to the filtrate side thereof, while withdrawing all or part of the raw water and filtrate in the module as a residue of the filtration cycle or alternatively after withdrawal of said raw water and filtrate.

2. A process according to Claim 1 wherein water is introduced into the filtration device from the raw water side if cooling takes place after steam has been passed, thereby preventing the filtrate side of the filtration device from becoming negative in pressure.

3. A process according to Claim 2, wherein the water temperature is 80°C or higher.

4. A process according to Claim 1, wherein a sterilized gas is introduced into the filtration device from the filtrate side if cooling takes place after steam has been passed, thereby preventing the filtrate side of the filtration device from becoming negative in pressure.

5. A process according to Claim 4, wherein the sterilized gas has a pressure of 4.9 to 9.8 x 10⁴ Pa (0.5 to 1 Kg/cm²).

6. A process according to any one of Claims 1 to 5 wherein the steam has a temperature of 121 to 125°C.

7. A process according to any one of Claims 1 to 6 wherein the steam has a pressure of 9.8 to 14.7 x 10⁴ Pa (1 to 1.5 Kg/cm²).

8. A process according to any one of Claims 1 to 7 wherein the sterilization is carried out for 0.5 to 1 hour.

**Revendications**

1. Procédé de stérilisation d'un appareil de filtration, qui consiste (a) à prévoir un boîtier de module contenant un faisceau de fibres creuses en un polymère résistant à la chaleur, ayant une température de déformation sous l'effet de la chaleur de 70°C ou supérieure à 70°C sous une charge de 182 x 104 Pa (18,6 kg/cm²) et un diamètre de pores inférieur à 0,45 μm, les fibres étant scellées ensemble et aux surfaces intérieures du boîtier adjacent à chaque extrémité du faisceau par un matériau scellement tel que la différence de coefficients moyens de dilatation linéaire entre le matériau de scellement et le boîtier du module, à une température comprise entre 20 et 121°C, soit de 7 x 10⁻⁵ °C ou soit inférieure à cette valeur; (b) à faire passer de la vapeur d'eau du côté de l'eau brute du module à son côté de filtrat, tout en soutirant tout ou partie de l'eau brute et du filtrat du module sous la forme d'un résidu du cycle de filtration ou, en variante, après avoir soutiré l'eau brute et le filtrat.

2. Procédé suivant la revendication 1, qui consiste à introduire de l'eau dans l'appareil de filtration par le côté de l'eau brute, si un refroidissement a lieu après le passage de la vapeur d'eau, de manière à empêcher le côté du filtrat de l'appareil de filtration de prendre une pression négative.

3. Procédé suivant la revendication 2, dans lequel la température de l'eau est de 80°C ou est supérieure à 80°C.

4. Procédé suivant la revendication 1, qui consiste à introduire un gaz stérilisé dans l'appareil de filtration par le côté du filtrat, si un refroidissement a lieu après le passage de la vapeur d'eau, de manière à empêcher le côté du filtrat de l'appareil de filtration de prendre une pression négative.

5. Procédé suivant la revendication 4, dans lequel le gaz stérilisé a une pression de 4,9 à 9,8 x 10⁴ Pa (0,5 à 1 kg/cm²).

6. Procédé suivant l'une quelconque des revendications 1 à 5, dans lequel la vapeur d'eau a une température de 121 à 125°C.

7. Procédé suivant l'une quelconque des revendications 1 à 6, dans lequel la vapeur d'eau a une pression de 9,8 à 14,7 x 10⁴ Pa (1 à 1,5 kg/cm²).

8. Procédé suivant l'une quelconque des revendications 1 à 7, qui consiste à effectuer la stérilisation pendant une demi-heure à une heure.

## Patentansprüche

1. Verfahren zum Sterilisieren einer Filtervorrichtung, welches umfaßt: (a) das Bereitstellen eines Modulgehäuses, das ein Bündel von Hohlfasern aus hitzebeständigem Polymer mit einer Wärmeverformungstemperatur von 70°C oder höher unter einer Last von 182 x $10^4$ Pa (18,6 kg/cm²) und einem Porendurchmesser von weniger als 0,45 μm enthält, wobei die Fasern in der Nähe jedes Endes des Bündels miteinander und gegen die inneren Oberflächen des Gehäuses durch ein Dichtungsmaterial in der Weise abgedichtet sind, daß die Differenz zwischen dem durchschnittlichen linearen Ausdehnungskoeffizienten des Dichtungsmaterials und des Modulgehäuses bei einer Temperatur von 20 - 121°C 7 x $10^{-5}/°C$ oder weniger beträgt; (b) Hindurchleiten von Dampf von der Rohwasserseite des Moduls zu dessen Filtratseite unter Abziehen des gesamten oder eines Teils des Rohwassers und des in dem Modul als ein Rückstand des Filtrationszyklus befindlichen Filtrats oder alternativ nach dem Abziehen des Rohwassers und Filtrats.

2. Verfahren nach Anspruch 1, wobei Wasser in die Filtrationsvorrichtung von der Rohwasserseite her eingeführt wird, wenn Kühlung stattfindet, nachdem Dampf hindurchgeschickt worden ist, wodurch verhindert wird, daß auf der Filtratseite der Filtrationsvorrichtung der Druck negativ wird.

3. Verfahren nach Anspruch 2, wobei die Wassertemperatur 80°C oder mehr beträgt.

4. Verfahren nach Anspruch 1, wobei ein sterilisiertes Gas in die Filtrationsvorrichtung von der Filtratseite her eingeführt wird, wenn Kühlung stattfindet, nachdem Dampf hindurchgeschickt worden ist, wodurch verhindert wird, daß auf der Filtratseite der Filtrationseinrichtung der Druck negativ wird.

5. Verfahren nach Anspruch 4, wobei das sterilisierte Gas einen Druck von 4,9 bis 9,8 x $10^4$ Pa (0,5 bis 1 kg/cm²) hat.

6. Verfahren nach einem der Ansprüche 1 bis 5, wobei der Dampf eine Temperatur von 121 bis 125°C hat.

7. Verfahren nach einem der Ansprüche 1 bis 5, wobei der Dampf einen Druck von 9,8 bis 14,7 x $10^4$ Pa (1 bis 1,5 kg/cm²) hat.

8. Verfahren nach einem der Ansprüche 1 bis 7, wobei die Sterilisation für 0,5 bis 1 Stunde durchgeführt wird.

# F I G. 1

# FIG. 2